# FASCICULE DE BREVET EUROPEEN

(11) **EP 0 594 506 B1**
(45) Date de publication et mention de la délivrance du brevet: **15.04.1998**
(21) Numéro de dépôt: 93402580.0
(22) Date de dépôt: 20.10.1993
(51) Int. Cl.: G01N 33/538, G01N 33/555, G01N 33/80, G01N 33/559

(54) **Procédé d'immunodiagnostic en matrice d'affinité poreuse avec particules sensibilisées et dispositif pour sa mise en oeuvre**
Immunodiagnostisches Verfahren mit Hilfe einer porösen Affinitätsmatrix mit sensibilisierten Partikeln und Vorrichtung dafür
Immunodiagnostic method using porous affinity matrix with sensitized particles and device therefore

(30) Priorité: 21.10.1992 FR 9212605
(43) Date de publication de la demande: 27.04.1994
(73) Titulaire: PASTEUR SANOFI DIAGNOSTICS, 92430 Marnes La Coquette (FR)
(72) Inventeur: Pernelle, Michel, F-91430 Igny (FR)
(74) Mandataire: Polus, Camille

(56) Documents cités:
- EP-A- 0 194 212
- EP-A- 0 363 510
- EP-A- 0 403 679
- WO-A-89/05456
- WO-A-91/16116
- WO-A-92/05442
- DE-A- 2 737 491
- FR-A- 2 660 437
- FR-A- 2 673 472

## Description

La présente invention concerne un procédé d'immunodiagnostic en phase solide dans lequel la formation du complexe immun se traduit par une localisation particulière de microparticules dans une matrice d'affinité.

L'utilisation dans les immunoessais de particules, portant un antigène ou un anticorps spécifique d'un complexe immun dont l'un des 2 éléments est à déterminer, est bien connue que ce soit pour des particules naturelles comme les érythrocytes ou des microbilles d'un polymère, comme les latex de synthèse, éventuellement magnétiques.

La formation d'agglutinats ou l'immunoadhérence de ces particules sur une phase solide d'immunoaffinité convenable sont deux moyens de visualiser la réaction immunologique.

Les groupages et phénotypages sanguins, la recherche des agglutinines irrégulières dans le sérum font généralement appel à des techniques d'agglutination plus ou moins complexes selon le pouvoir agglutinant des anticorps. Ainsi, dans certains cas, on devra introduire dans le milieu réactionnel contenant le sérum et les hématies, des macromolécules comme l'albumine ou le dextran ou opérer en plusieurs étapes, comme dans le test de Coombs, c'est-à-dire séparation des hématies après incubation avec le sérum, lavage, introduction dans un milieu contenant une antiglobuline humaine puis centrifugation.

Dans le brevet EP-A-194 212, on décrit un procédé pour la mise en évidence d'une agglutination érythrocytaire dans lequel le mélange sérum-hématies incubé est soumis à une centrifugation douce à travers un milieu gélifié auquel est mélangé, si l'agglutination n'est pas spontanée, un anticorps ou un autre réactif, de telle sorte que les agglutinats alors formés restent vers le haut du gel, tandis que les érythrocytes ne portant pas le complexe antigène-anticorps se déposent au fond du tube. Dans la demande EP-A-305 337, le milieu est constitué de microparticules de taille et densité convenables au lieu d'un gel.

Dans FR-A-2 673 472, le mélange sérumhématies incubé est placé en haut d'une microcolonne ouverte contenant une phase inerte convenable dans laquelle on fait passer un liquide, de telle sorte que les agglutinats formés restent dans le haut de ladite colonne.

Dans ces deux types de techniques, on sépare les particules agglutinées de celles restées libres grâce à leur différence de taille, les particules les plus petites migrant plus facilement, sous l'action de la force due à la centrifugation ou à la percolation, dans la matrice de porosité convenable constituée d'un gel ou de microparticules en suspension.

Dans le procédé de la présente invention, la séparation des particules de départ de celles portant un complexe immun - qui peuvent être agglutinées ou non - n'est pas due à leur différence de taille mais d'immuno-réactivité ; l'influence de l'intensité et de l'orientation de la force appliquée sur les particules sur la sensibilité et la reproductibilité du test est ainsi plus faible, et le choix des caractéristiques physiques de la matrice est moins critique.

Le procédé d'immunodiagnostic selon l'invention consiste à :
a) mettre en contact des particules portant éventuellement un antigène - ou un anticorps - avec un milieu liquide susceptible de contenir un anticorps - ou un antigène - correspondant, pour former un complexe immun ;
b) faire passer sous l'action d'une centrifugation le milieu réactionnel précédent à travers une matrice poreuse, sur laquelle est immobilisée une substance susceptible de se lier à l'anticorps - ou à l'antigène - éventuellement dans le milieu liquide ; et
c) déterminer la présence éventuelle de particules portant le complexe immun, fixées dans la matrice par la substance qui y est immobilisée, en observant la position des particules dans cette matrice.

Selon le cas, c'est l'antigène - ou l'anticorps - à déterminer qui sera immobilisé sur la particule ou sera libre dans le milieu liquide.

Les particules peuvent être des cellules sanguines, érythrocytes ou lymphocytes par exemple, ou des microbilles de diamètre de l'ordre du micron de densité suffisante pour qu'elles sédimentent dans les conditions de l'essai et qui seront constituées d'un polymère naturel réticulé ou synthétique : on en trouve différents exemples dans le commerce, adaptés pour une utilisation en immunoessai.

Les antigènes - ou anticorps - seront immobilisés sur les particules par les moyens bien connus de l'homme du métier, par liaison chimique ou par adsorption, lorsqu'ils ne sont pas naturellement présents comme sur les cellules sanguines. On pourra notamment se référer à l'ouvrage "Antibodies-Ed Harlow, David, Lane (1988)" qui décrit des techniques d'immobilisation des protéines.

Comme la répartition des particules dans la matrice est étudiée à l'oeil nu ou à l'aide d'un photomètre, celle-ci doit être sensiblement transparente et les particules colorées, comme le sont les érythrocytes naturellement ; on peut aussi utiliser des particules pratiquement incolores - comme les lymphocytes - dans un milieu coloré mais transparent, ou les colorer préalablement.

Le milieu liquide peut être un liquide biologique, sang total, sérum, urine, ou une suspension cellulaire, éventuellement dilués, dans les conditions bien connues de l'homme du métier, avec des tampons physiologiques ou les solutions habituelles qui accélèrent la formation des complexes immuns telles que la solution basse force ionique.

On peut utiliser le procédé de l'invention pour la recherche des antigènes naturellement présents sur les érythrocytes des systèmes Rhésus, Lutheran, Kell, Duffy et autres, ou pour celle d'anticorps, et notamment ceux dits irréguliers, qui ne sont présents dans le sérum humain qu'après une immunisation, comme dans l'alloimmunisation foetomaternelle ou transfusionnelle ; le typage HLA des lymphocytes est un autre exemple d'application.

Dans les domaines de la bactériologie et de la virologie, on utilisera des particules synthétiques ou des érythrocytes, sur lesquels on aura fixé un anticorps ou un antigène qui pourront se complexer à la substance à étudier.

Après la première étape d'incubation, au cours de laquelle se forme le complexe immun si la substance à doser est présente, le milieu réactionnel est introduit sur une matrice poreuse dans laquelle est immobilisée une substance qui a la capacité de fixer l'antigène - ou l'anticorps - du milieu liquide.

La matrice peut être placée dans un microtube ou dans les cupules d'une plaque de microtitration ; elle peut se présenter sous forme d'une masse gélifiée ou sous forme de microbilles, notamment d'agarose ou de polymères acryliques, de diamètre compris entre 40 et 200 µm, dans un tampon physiologique ou une solution favorisant la formation du complexe immun. L'état physique, et notamment la porosité de la matrice, sera tel que les particules puissent y circuler sous l'action d'une force dirigée à peu près selon l'axe du tube, sans que cela entraîne une déformation sensible de ladite matrice.

Ladite force peut résulter d'une centrifugation ou d'une augmentation de pression au-dessus de la matrice.

On préfère la centrifugation, mais les conditions de celle-ci ne sont pas critiques, contrairement à celles du procédé décrit dans le brevet européen EP-A-305 337 ; il suffit en effet d'éviter que les particules ne soient entraînées trop rapidement vers le fond du tube, ce qui gênerait la formation de liaisons spécifiques avec la matrice pour celles qui ont été sensibilisées pendant la phase d'incubation, c'est-à-dire qui portent le complexe immun.

La matrice peut porter une substance comme les protéines A ou G, une anti-immunoglobuline IgG humaine ou ses fragments Fab ou F(ab)'₂ lorsqu'un antigène est immobilisé sur les particules pour un diagnostic en hématologie, sérologie ou virologie.

Lorsqu'un anticorps est immobilisé sur les particules, la substance sur la matrice est un autre anticorps, monoclonal ou polyclonal, qui reconnaît l'antigène du milieu liquide.

Les moyens de fixation de la substance à la matrice sont bien connus de l'homme du métier, que ce soit par liaison chimique, ou par liaison de type immunologique, par exemple par l'intermédiaire de la protéine A ; on pourra par exemple utiliser des gels où est fixée la protéine A telle que le Sepharose CL4B-protein A de chez Pharmacia, ou IPA-300 et 400 de chez Repligen. On peut aussi utiliser des gels tels que Sephadex G200 ou Sepharose 6MB (BrCN), sur lesquels on fixe - selon la méthode préconisée par le fournisseur Pharmacia - de la protéine A ou G ou une anti-IgG. Parmi les microbilles, on peut citer la n-protein A Avidgel de chez Bioprobe Int.

En général, ces produits commerciaux sont passivés préalablement à leur emploi pour éviter les fixations non spécifiques.

La matrice peut être aussi composée de deux couches superposées, la couche inférieure ne contenant pas de substance affine.

Sous l'action de la force appliquée, lorsque les particules introduites dans la matrice poreuse après incubation dans le milieu liquide ne portent pas de complexe immun, elles la traverseront et se réuniront au fond du tube ou de la cupule de la plaque de microtitration au bout d'un temps qui sera fonction du matériel utilisé et des conditions opératoires, mais que l'homme du métier pourra déterminer sans difficulté. Par contre, les particules sensibilisées, c'est-à-dire portant le complexe immun, seront arrêtées dès qu'elles rencontreront la substance affine fixée sur la matrice ; on observera alors une tranche colorée vers le haut du tube dont l'épaisseur et la distance à la surface de la matrice seront fonction des conditions particulières du procédé fixées par le technicien.

Le procédé selon l'invention est particulièrement simple à mettre en oeuvre lorsque les deux étapes d'incubation et de révélation peuvent être effectuées dans un même dispositif. A cette fin, on peut utiliser un microtube en verre ou en matière plastique évasé vers le haut comme ceux décrits dans EP-A-305 337 précédemment cité. Mais d'autres dispositifs conviennent.

Ainsi, on a constaté que le milieu d'incubation ne traversait pas spontanément, sous l'effet de la seule gravité, un filtre dont les diamètres des pores ne sont pas trop grands et qui est composé d'un matériau hydrophobe tel que le polyéthylène, le polypropylène, fritté ou tissé ; le milieu traverse par contre ledit filtre lorsqu'il est soumis à une force, telle que celle utilisée pour le faire passer à travers la matrice poreuse.

Aussi, un autre objet de l'invention est un récipient utilisable pour la mise en oeuvre du procédé selon l'invention, caractérisé en ce qu'il contient un filtre en matériau hydrophobe de diamètre de pores compris entre 5 et 150 µm, de préférence entre 40 et 50 µm et dont l'épaisseur est de l'ordre du mm, ledit filtre étant situé entre la zone supérieure d'incubation et la partie inférieure contenant la matrice d'affinité, ces deux zones pouvant être séparables.

On trouve dans le commerce des filtres convenables, tel que le polyéthylène fritté d'Isolab (USA) ou le polypropylène tissé, dénommé Scrynel de Polylabo (FR).

Le récipient peut se présenter sous forme d'un tube à essais pour microdosages qui comporte une partie supérieure cylindrique de diamètre compris entre 4 et 10 mm et de hauteur comprise entre 2 et 5 cm, et une partie inférieure, de préférence conique, de 0,5 à 2 cm de hauteur, qui contiendra la matrice d'affinité. Le filtre de 0,5 mm à 5 mm d'épaisseur, selon sa rigidité, de diamètre au plus égal à celui de la partie cylindrique du tube, sera placé au-dessus de la matrice ou des matrices, de préférence en laissant entre le filtre et la (les) matrice(s) un volume proche de celui du milieu d'incubation.

On peut utiliser une couche plus fine de matrice d'affinité lorsqu'elle est disposée sur une couche de matrice neutre d'au moins la même épaisseur, et de préférence d'une épaisseur de 2 à 3 fois celle de la matrice d'affinité.

On peut également remplacer le filtre par une couche d'une huile minérale, végétale, ou de synthèse, de préférence transparente, de densité inférieure à celle de la matrice, de viscosité suffisante pour que le milieu d'incubation ne la franchisse pas spontanément. On peut par exemple utiliser l'huile de paraffine commercialisée par La Coopération Française (réf. 2164). Le filtre est alors posé sur la matrice.

Pour effectuer plusieurs dosages simultanément, on peut également solidariser plusieurs récipients^placés l'un à côté de l'autre à l'aide d'un support en carton ou d'un portoir en matière plastique, avec lequel éventuellement les tubes ne feront qu'une seule pièce, l'homme du métier étant à même de réaliser diverses solutions en tenant compte des moyens nécessaires à l'application de la force sur les particules.

Lorsque la matrice est distribuée dans les cupules d'une plaque de microtitration, de préférence dans des cupules à fond conique d'au moins 1 cm de profondeur, on préfère, pour que la zone d'incubation ait un volume suffisant, utiliser un récipient annexe adapté pour éviter le transvasement du milieu incubé. Ainsi, un autre dispositif selon l'invention est constitué d'une plaque de microtitration dont les cupules contiennent une matrice d'affinité, éventuellement avec une matrice neutre disposée sous la matrice d'affinité, ainsi que d'une plaque de mêmes type et format, à placer dessus, comportant aussi une série de petites cavités, de préférence de forme cylindrique ou légèrement conique, de dimensions correspondant à celle de la microplaque, le fond de ces cavités étant muni d'un filtre en matériau hydrophobe, de diamètre de pores compris entre 5 et 150 µm et d'épaisseur de l'ordre du mm.

La surplaque peut être solidarisée à la microplaque par un ensemble de clips fixés sur les bords, ou les cavités de la surplaque peuvent être engagées dans les cupules sur une faible hauteur, bloquant ainsi l'une sur l'autre ; les cavités et les cupules doivent alors avoir des sections de dimensions adaptées.

Dans ce qui suit, des exemples du procédé de l'invention sont décrits ; ils ont été effectués dans des tubes à fond conique, dits tubes à sédimentation, en matière plastique de hauteur totale de 4,5 cm, de diamètre intérieur de 2 mm et de hauteur de 0,7 cm dans la partie cylindrique inférieure, puis s'évasant sur une hauteur de 0,3 cm jusqu'à un diamètre de 5 mm, et se terminant par une partie supérieure cylindrique de 5 mm de diamètre jusqu'à la hauteur totale de 4,5 cm, de telle sorte que le milieu d'incubation ne s'écoule pas spontanément sur la matrice placée préalablement dans la partie cylindrique inférieure. Ces tubes sont commercialisés par Starstedt (Ivry - France) sous la référence 72702.

### EXEMPLE 1

On introduit dans le tube à sédimentation 20 µl de gel neutre préparé à partir de Sephadex ® G100 Superfine réhydraté, puis passivé par mise en suspension dans une solution d'albumine bovine à 3% dans un tampon phosphate salin de pH 7,2 à raison de 0,5 g de poudre pour 10 ml de solution (comme préconisé par le fournisseur).

On introduit ensuite 10 µl de gel d'affinité constitué d'un gel d'agarose à 6% sur lequel est fixé par liaison covalente de la protéine A recombinante, réhydraté et passivé dans les mêmes conditions que le gel neutre. Ce gel est commercialisé par Repligen Corporation sous la référence IPA-300.

Une centrifugation d'une minute à 150 g permet de tasser le gel et d'éliminer les bulles d'air éventuelles.

On dispose ensuite 30 µl du sérum plus ou moins dilué, à tester, et 50 µl d'une suspension d'érythrocytes de spécificité connue, à 0,8% d'hématocrite dans une solution de basse force ionique.

Après 10 minutes d'incubation à 37°C, le tube est centrifugé durant 8 minutes à 250 g et l'image obtenue observée : la présence d'un point rouge au fond du tube traduit l'absence de formation de complexe immun, et donc une réaction négative, tandis que la présence d'une bande rouge dans la partie supérieure de la matrice traduit une réaction nettement positive.

On a ainsi étudié divers sérums de patients contenant, de façon connue, des agglutinines irrégulières, en utilisant les érythrocytes correspondants du commerce.

Dans le tableau I sont indiqués les titres, c'est-à-dire l'inverse de la plus grande dilution du sérum dans une solution aqueuse saline à 9% contenant de l'albumine bovine à 6%, qui donne un résultat encore positif, ainsi que les titres obtenus avec la technique de Coombs classique pour le même échantillon.

| **Sérum avec** | **Exemple : titre** | **Test de Coombs : Titre** |
|---|---|---|
| anti-E | 32 | 8 |
| anti-S | 64 | 16 |
| anti-Fya | 64 | 16 |
| anti-D | 40 000 | 5 000 |

### EXEMPLE 2

On mélange 4 volumes de Sephadex ® G100 Superfine et 1 volume de gel IPA-300 passivés comme à l'exemple précédent et on en introduit 30 µl dans un microtube.

On opère ensuite comme à l'exemple précédent mais avec un sérum test anti-K₁, monoclonal, et une suspension d'érythrocytes 2 à 0,8% d'un patient dans une solution basse force ionique. Une réaction positive est observable jusqu'à un titre de 1 000 avec la technique de Coombs classique, et un titre de 8 000 dans les conditions du présent exemple.

### EXEMPLE 3

On dispose dans un microtube 20 µl de gel Sephadex ® G100 Superfine, réhydraté et passivé comme à l'exemple 1, puis 10 µl de Sepharose 6MB (-CNBr/activated) commercialisé par Pharmacia, sur lequel on a fixé à raison de 2 mg/ml de gel, des IgG anti-Fc humaines selon un procédé préconisé par le fournisseur.

On introduit ensuite dans la partie évasée du tube 30 µl de sérum, plus ou moins dilué comme à l'exemple 1, et 50 µl de suspension d'hématies de spécificité connue.

Après 10 minutes d'incubation à 37°C et une centrifugation de 8 minutes à 150 g, on observe l'image formée dans la matrice gélifiée.

Dans le cas d'un sérum anti-D, le titre le plus faible décelable est 80 000, alors qu'il est de 5 000 dans la technique de Coombs classique.

Pour un sérum anti-S, on obtient respectivement les titres de 128 et 16.

### EXEMPLE 4

On opère comme à l'exemple 3, mais le gel d'affinité est un gel Sepharose CL4B-(Fab anti-IgG humaine) à 2 mg d'anti-IgG / ml de gel, commercialisé par Pharmacia. Après 8 minutes de centrifugation à 250 g, le titre pour du sérum anti-D est 40 000 pour 5 000 en technique de Coombs et pour un sérum anti-S de 64 et 16.

## Revendications

1. Procédé d'immunodiagnostic comprenant les étapes consistant à :
a) mettre en contact des particules portant éventuellement un antigène - ou un anticorps - avec un milieu liquide susceptible de contenir un anticorps - ou un antigène - correspondant, pour former un complexe immun ;
b) faire passer en centrifugeant le milieu incubé précédent à travers une matrice poreuse, sur laquelle est immobilisée une substance susceptible de se lier à l'anticorps - ou à l'antigène - éventuellement présent dans le milieu liquide ; et
c) observer la position des particules dans ladite matrice.

2. Procédé selon la revendication 1, caractérisé en ce que les particules sont des érythrocytes.

3. Procédé selon l'une des revendications précédentes, caractérisé en ce que les particules portent un antigène et en ce que la substance susceptible de se lier à l'anticorps est une immunoglobuline anti-IgG, ou ses fragments Fab et F(ab)'₂.

4. Procédé selon l'une des revendications 1 à 3, caractérisé en ce que les particules portent un antigène et en ce que la substance immobilisée est la protéine A ou la protéine G.

5. Procédé selon l'une des revendications 1 à 3, caractérisé en ce que les particules portent un anticorps et en ce que la substance immobilisée est un anticorps monoclonal ou polyclonal spécifique de l'antigène.

6. Procédé selon la revendication 1, caractérisé en ce que les particules sont des érythrocytes, et que l'anticorps du milieu liquide est dirigé contre un antigène d'érythrocyte.

7. Dispositif pour la mise en oeuvre d'un procédé selon l'une des revendications 1 à 6, caractérisé en ce qu'il est constitué d'un tube pour microdosages dont le fond contient la matrice poreuse, surmontée d'un filtre en matériau hydrophobe de diamètre de pores compris entre 5 et 150 µm et d'épaisseur de l'ordre du mm.

8. Dispositif pour la mise en oeuvre d'un procédé selon l'une des revendications 1 à 6, caractérisée en ce qu'il est constitué d'une plaque de microtitration, dont les cupules contiennent la matrice poreuse, et d'une surplaque de même format à cavités sensiblement cylindriques dont l'axe coïncide avec celui des cupules et dont le fond est constitué d'un filtre en matériau hydrophobe de diamètre de pores compris entre 5 et 150 µm et d'épaisseur de l'ordre du mm.

## Patentansprüche

1. Immunodiagnostisches Verfahren umfassend die folgenden Stufen:
a) Inkontaktbringen von Teilchen, welche gegebenenfalls ein Antigen - oder einen Antikörper - tragen, mit einem flüssigen Medium, welches möglicherweise einen entsprechenden Antikörper - oder ein entsprechendes Antigen - enthält zur Bildung eines Immunkomplexes;
b) Hindurchführen des in der obigen Weise inkubierten Mediums unter Zentrifugieren durch eine poröse Matrix, auf welcher eine Substanz immobilisiert ist, welche an den gegebenenfalls in dem flüssigen Medium vorhandenen Antikörper - oder Antigen - zu binden vermag; und
c) Beobachten der Position der Teilchen in der genannten Matrix.

2. Verfahren nach Anspruch 1, dadurch gekennzeichnet. daß die Teilchen Erythrocyten sind.

3. Verfahren nach einem der vorhergehenden Ansprüche, dadurch gekennzeichnet, daß die Teilchen ein Antigen tragen und die Substanz, die sich mit dem Antikörper zu verbinden vermag, ein Anti-IgG-Immunoglobulin ist oder seine Fragmente Fab und F(ab)'₂.

4. Verfahren nach einem derAnsprüche 1 bis 3, dadurch gekennzeichnet, daß die Teilchen ein Antigen tragen und die immobilisierte Substanz Protein A oder Protein G ist.

5. Verfahren nach einem der Ansprüche 1 bis 3, dadurch gekennzeichnet, daß die Teilchen einen Antikörper tragen und die immobilisierte Substanz ein für das Antigen spezifischer monoklonaler oder polyklonaler Antikörper ist.

6. Verfahren nach Anspruch 1, dadurch gekennzeichnet, daß die Teilchen Erythrocyten sind und der Antikörper des flüssigen Mediums gegen ein Erythrocyten-Antigen gerichtet ist.

7. Vorrichtung zur Durchführung eines Verfahrens nach einem der Ansprüche 1 bis 6, dadurch gekennzeichnet, daß sie aus einem Mikrodosierungsröhrchen gebildet ist, dessen Boden die poröse Matrix enthält, oberhalb der ein Filter aus einem hydrophoben Material angeordnet ist mit einem Porendurchmesser zwischen 5 und 150 µm und einer Dicke im mm-Bereich.

8. Vorrichtung zur Durchführung eines Verfahrens nach einem der Ansprüche 1 bis 6, dadurch gekennzeichnet, daß sie aus einer Mikrotiterplatte gebildet ist, deren Vertiefungen die poröse Matrix enthalten und einer Deckplatte gleichen Formats mit im wesentlichen zylindrischen Öffnungen, deren Achse jener der Vertiefungen entspricht und deren Boden aus einem Filter aus einem hydrophoben Material mit einem Porendurchmesser zwischen 5 und 150 µm und einer Dicke im mm-Bereich gebildet ist.

## Claims

1. Immunodiagnostic process comprising the steps of:
a) bringing particles which may contain an antigen - or an antibody - into contact with a liquid medium capable of containing a corresponding antibody - or antigen - in order to form an immune complex;
b) passing the abovementioned incubated medium, by centrifuging, through a porous matrix on which is fixed a substance capable of binding to the antibody - or antigen - which may be present in the liquid medium; and
c) observing the position of the particles in said matrix.

2. Process according to claim 1, characterised in that the particles are erythrocytes.

3. Process according to one of the preceding claims, characterised in that the particles carry an antigen and in that the substance capable of binding to the antibody is an anti-IgG immunoglobulin, or the Fab and F(ab)'₂ fragments thereof.

4. Process according to one of claims 1 to 3, characterised in that the particles carry an antigen and in that the fixed substance is protein A or protein G.

5. Process according to one of claims 1 to 3, characterised in that the particles carry an antibody and in that the fixed substance is a monoclonal or polyclonal antibody specific to the antigen.

6. Process according to claim 1, characterised in that the particles are erythrocytes, and in that the antibody of the liquid medium is directed against an erythrocyte antigen.

7. Apparatus for carrying out a process according to one of claims 1 to 6, characterised in that it consists of a microtitration tube the base of which contains the porous matrix, surmounted by a filter made of hydrophobic material having a pore diameter of between 5 and 150 µm and having a thickness of the order of 1 mm.

8. Apparatus for carrying out a process according to one of claims 1 to 6, characterised in that it consists of a microtitre plate the wells of which contain the porous matrix, and a top plate of the same size with substantially cylindrical cavities the axis of which coincides with that of the wells and the base of which is made up of a filter of hydrophobic material having a pore diameter of between 5 and 150 µm and having a thickness of the order of 1 mm.
